# EUROPEAN PATENT SPECIFICATION

(11) **EP 1 748 700 B1**
(45) Date of publication and mention of the grant of the patent: **30.01.2008**
(21) Application number: 05718511.8
(22) Date of filing: 20.04.2005
(51) Int. Cl.: A23G 1/00, C07B 63/00

(54) **PROCESS FOR PRODUCING COCOA POLYPHENOL CONCENTRATE**
VERFAHREN ZUR HERSTELLUNG EINES KAKAOPOLYPHENOL-KONZENTRATS
PROCEDE PERMETTANT DE PRODUIRE UN CONCENTRAT DE POLYPHENOLS DE CACAO

(30) Priority: 24.05.2004 GB 0411556
(43) Date of publication of application: 07.02.2007
(73) Proprietor: Natraceutical S.A., 46930 Valencia (ES)
(72) Inventor: PONS-ANDREU, José-Vicente, E-46930 Quart de Poblet (ES); CIENFUEGOS-JOVELLANOS, Elena, E-46930 Quart de Poblet (ES); IBARRA, Alvin, E-46930 Quart de Poblet (ES)
(74) Representative: Torras Toll, Jorge
(86) International application number: PCT/IB2005/001075
(87) International publication number: WO 2005/115160

(56) References cited:
- EP-A- 0 906 761
- EP-A- 1 026 164
- WO-A-02/14251
- US-A- 6 015 913
- US-B1- 6 265 593
- PATENT ABSTRACTS OF JAPAN vol. 2000, no. 03, 30 March 2000 (2000-03-30) & JP 11 346656 A (MORINAGA & CO LTD), 21 December 1999 (1999-12-21)

## Description

The present invention relates to processes for obtaining cocoa products which are rich in cocoa polyphenols where the starting materials are unfermented cocoa beans. More specifically, the present invention discloses a process for producing liquid and powdered cocoa polyphenol concentrate from unfermented cocoa beans.

At present there is a great interest in cocoa polyphenols not only because of their contribution to flavour, but also for their antioxidant activity and possible beneficial health effects. The total polyphenol content in fresh unfermented defatted and dried cocoa beans can vary in the range from 12 to 20 wt%. The principal polyphenol compounds present are monomeric flavonoids such as (+)-catechin, (-)-epicatechin and oligomeric flavonoids such as proanthocyanidins, of which half are dimers (B1 - B8). Other compounds which have been identified are quercetin and quercetin glycosides, quercetrin, luteolin, apigenin and pcoumaric, cafeic, chlorogenic acids and others.

Obtaining these from cocoa has already been addressed in, e.g., US 6 015 913; EP 1 026 164; and EP 0 906 761.

Manufacturing cocoa involves fermentation, during which many biochemical reactions occur in order to develop the components and precursors of chocolate colour and flavour. Fermentation processes, among others, reduce the astringency, acidity and bitterness of cocoa.

During the fermentation process, fresh cocoa beans are heaped in a pile or in a wooden box, typically for 5 days. Fermentation can be divided into two principal stages, anaerobic fermentation and aerobic fermentation. During anaerobic fermentation, the cocoa flavour precursors are formed. Sugar from the fruit pulp is broken down into the reducing sugars, fructose and glucose, and proteins are broken down into peptides and free amino acids. These products form the precursors for the Maillard reaction in which the characteristic cocoa and chocolate flavours are developed during the drying and roasting of the beans. Anaerobic fermentation optimises the conditions for enzymatic browning, a reaction carried out by Polyphenol. Oxidase (PPO). Normally, aerobic fermentation begins after the second day of fermentation. In this stage, PPO catalyses the oxidation of phenols into ortho-quinones, which are highly reactive compounds. Once formed, ortho-quinones undergo spontaneous polymerization to produce high-molecular-weight compounds or brown pigments (melanins). These melanins may in turn react with amino acids and proteins intensifying the brown colour. These high molecular weight condensed polymers become increasingly insoluble as the degree of polymerization increases. Furthermore, they have less bioavailability than their precursors. Conditions during aerobic fermentation are ideal for reactions with PPO, and the high oxygen uptake during drying further promotes fomentation.

Many studies have focused on either inhibiting or preventing PPO activity in foods. Various techniques have been developed over the years to inhibit these undesirable enzymatic activities. Several of these techniques can be applied in order to prevent enzymatic browning. For example in the paper titled " The manufacture of cocoa polyphenols as healthy supplements in food products", Kattenberg et al. describe the use of microwave heating to prevent PPO activity in unfermented beans. The use of microwaves is, however, expensive as specialised equipment is required. Furthermore, when using microwave treatment, it is difficult to control the temperature within the unfermented beans.

Unfermented cocoa beans have neither the same organoleptic profile, nor the same polyphenol profile as fermented cocoa beans. Unfermented beans have a very bitter and astringent taste with little apparent chocolate flavour.

During cocoa processing, the content of flavonols (monomers) present in the original unfermented raw cocoa beans depends largely on post-harvest handling and processing techniques. Both the fermentation and alkali steps in cocoa powder and chocolate manufacture can greatly reduce the monomer content. During fermentation the total number of phenols in cocoa are reduced to 30% of their initial value and (-)-epicatechin, the principal substrate of cocoa polyphenol oxidase (PPO), is reduced by 90% with a proportional increase in catechin content.

The fermentation and drying processes alter the content and composition of polyphenolic compounds. Fermented cocoa beans contain a smaller amount of low molecular weight phenols and a higher content of condensed polyphenols. Condensed polyphenolics are involved in protein-phenol interactions and may contribute to the low digestibility and poor biological value of phenols and cocoa proteins.

Studies have demonstrated that cocoa polyphenols increase the resistance of LDL-C oxidation and plasma antioxidant capacity, modulate platelet activity, increase nitric oxide synthesis and vasodilatation, modulate eicosanoid synthesis, protect against peroxynitrite and modulate the immune system.

The polyphenols that are most abundant in our diet are not necessarily the most beneficial, either because they are poorly absorbed from the intestine and rapidly eliminated, or because their metabolites have a low intrinsic activity. Monomeric flavonols are better absorbed than polymerized proanthocyanidins. Several studies show that the proanthocyanidins are not absorbed in the stomach and so their action is restricted to the intestine. Additionally, monomeric flavanols, such as catechins and epicatechins, have more antioxidant activity in both *in vitro* and *in vivo* studies regarding plasma.

It is therefore clear that there is a need for a simple and economical process whereby polyphenols can be extracted from unfermented cocoa beans wherein the polyphenols have not undergone polymerization as catalysed by the polyphenol oxidase enzyme.

The present invention provides a process in which cocoa polyphenol concentrate is obtained comprising the steps of:
a) subjecting unfermented cocoa beans to a blanching step in water at a temperature in the range from 85-100°C for a time period in the range from 3 to 15 minutes to give unfermented cocoa beans with reduced polyphenol oxidase activity;
b) drying the unfermented cocoa beans with reduced polyphenol oxidase activity at a temperature of less than 85°C to obtain dried unfermented cocoa beans with a moisture content of no more than 15wt%;
c) subjecting the dried unfermented cocoa beans to a particle size reduction step to obtain dry unfermented cocoa bean intermediate whereby at least 99wt% of the intermediate has a particle size less than or equal to 300µm;
d) extracting polyphenols from the dry unfermented cocoa bean intermediate to give a cocoa polyphenol extract and extracted solids;
e) concentrating the cocoa polyphenol extract to yield a cocoa polyphenol concentrate wherein the concentration of polyphenol present is at least 10 wt%: and wherein the process further comprises a defatting step which is carried out prior to step d).

Where the term unfermented is used, it refers to cocoa beans that have been removed from the pod for a maximum of 5 hours. More preferably the cocoa beans have been removed from the pod for less than 2 hours. Fermentation is a process which occurs naturally after cocoa beans have been removed from the pod in warm and humid conditions. Being a natural process it occurs on a relatively slow timescale and thus a cocoa bean which has been removed from the pod for only 5 hours will be substantially unfermented. From a commercial point of view, it is difficult to find a source of such beans.

In one embodiment of the present invention, it may be preferably to include a step prior to step a) in order to maintain the unfermented state of the unfermented cocoa beans. Such a step can include, for example, immersing the unfermented cocoa beans in a solution of water and ascorbic acid.

The inclusion of step a), the blanching step, is essential in order to ensure that minimal polymerization of the polyphenols occurs and thus the high content of monomeric polyphenols present in the unfermented cocoa beans is preserved. The blanching step is carried out in water at a temperature in the range from 85-100°C. This temperature ensures that the polyphenol oxidase enzyme is denatured and thus inactivated. The blanching step should last for a time period in the range from 3 to 15 minutes. If the duration of the step were any longer then other undesirable non-enzymatic browning reactions such as the maillard reaction may occur.

The use of water in the blanching step is not conventional as it would be usual practice to avoid making the unfermented cocoa beans wet for the reason that any moisture added has to be removed in a subsequent step. The Applicants' have, however, found that the inclusion of a blanching step using water produces a beneficial result wherein a high proportion of the polyphenols present in the original unfermented cocoa bean are recovered.

Following step a), the unfermented cocoa beans with reduced polyphenol oxidase activity are dried.

The initial moisture content in a fresh unfermented bean is between 45 wt% and 55wt%. After drying, the final moisture content should be less than 15wt%, preferably less than 10wt%.

In one embodiment of the present invention, in areas where the weather is comparatively dry at harvest time, the unfermented cocoa beans with reduced polyphenol oxidase activity are dried in the sun. The beans are spread out in a layer of about 100 mm thick during sunlight on mats, trays or terraces. The beans are raked over at intervals and heaped up and protected at night or when it rains. The trays can, as is common in Central and South America, be whole floors with a roof or on wheels. These are pushed back over the floor at night so that several can be run under a roof, one above the others, to save space. In West Africa, the beans are spread on any suitable horizontal surface (i.e. a concentrate terrace or polyethylene sheet). The preferred method, which is common in Ghana, is to spread the beans on mats made of split bamboo, which are then placed on low wooden frames. The mats can be rolled up to protect the beans when it rains. This method has several advantages in that it is easier to sort the beans in order to remove defective beans and foreign material. Additionally, there is less risk of contamination compared with beans being dried at ground level. It usually takes about a week of sunny weather to dry the beans to 7 to 8wt% moisture content needed to prevent mould during storage.

Alternatively in step b), the unfermented cocoa beans with reduced polyphenol oxidase activity can be dried using heated-air dryers (i.e. intermittent dryers, batch internal-recirculant dryers, mix-flow dryers and cross flow dryers), unheated-air dryers, a combination of heated- and unheated- air dryers, commercial grain dryers (like malt dryers), batch fluidized bed dryers, contact dryers at normal pressure or under vacuum (i.e. vacuum tray, vacuum band, plate, thin film, drum, rotating batch, horizontally agitated, indirect rotatory vertical agitated), or dielectric dryers (i.e. radiofrequency and microwave).

In step b) the temperature in the bean should not exceed 85°C and should preferably be less than 70 °C, more preferably less than 50 °C. This is because cocoa polyphenol polymerisation occurs at temperatures above 70 °C. Therefore, it is necessary to maintain low temperatures during the drying process. When using dielectric dryers, an appropriate frequency must be selected to avoid polymerization. For example, in radio-frequency dryers, frequencies between 15 to 35 MHz are appropriate, and in the case of microwave dryers frequencies between 900 and 2.300 MHz can be used.

In the dried unfermented cocoa beans obtained in step b), a large proportion of the initial polyphenol content is preserved. This is due to both PPO inactivation and soft conditions inhibiting both degradation and polymerization of the polyphenols. The principal characteristic of these dried unfermented cocoa beans is their intense violet colour that reflects the stabilization of the polyphenol.

The polyphenol content in the samples can be measured using one of several methods. The most common method used is the Folin-Ciocalteau method, which gives the result expressed in wt% (Singleton et al., 1999). Additionally, other methodologies for the identification and quantification of the polyphenols profile can be use, for instance High Pressure Liquid Chromatography (HPLC) systems and mass spectrometer, LC-MS, HPLC coupled to UV detection, Supercritical Fluid Extraction (SFE), HPLC using double on-line detection by diode array spectrophotometry, mass spectroscopy (HPLC-DAS-MS) and also LC-NMR.

Optionally the dried unfermented cocoa beans obtained in step b) can then be cracked. Furthermore a winnowing process can be applied to the dried bean in order to separate the shells. This gives two products one with shell content and the other without the shell. The technology for these processes is currently available in the cocoa powder industry. The polyphenol content in the product without shell i.e. dried unfermented cocoa bits with shell is in the range from 3 to 10 wt%, and the polyphenol content in the product without shell i.e. dried unfermented cocoa bits with shell is in the range from 4 and 11 wt%.

After step b), the dry unfermented cocoa beans obtained are subjected to a particle reduction step c). Inclusion of the particle size reduction step is necessary in order to favour polyphenol extraction in the later extraction step d). Following step c), at least 99wt% of the dry unfermented cocoa bean intermediate produced has a particle size of less than or equal to 300µm, preferably less than 100µm, more preferably less than 80µm, for example 40µm.

In an embodiment of the present invention where the dry unfermented cocoa beans of step b) have not been defatted prior to step c), it is preferable that step c) employs the use of a cryogenic mill.

Cryogenic mills are unique in that other mills would be blocked up by the fat content of the dried unfermented cocoa beans when the temperature exceeds that of the melting point of the fat. Cocoa usually contains between 50 and 55% of fat, depending on the variety and the type of cocoa. This fat commonly known as cocoa butter has a melting point between 30 and 37 °C. Current milling technologies work in this range of temperature, melting the product within the machine and making operation extremely difficult. Nitrogen or carbonic acid liquid can be used as a refrigerant to lower the temperature of the milling process. The appropriate temperature for milling whole beans should be less than 0°C, preferably less than -5°C, more preferably less than -10 °C, for example -15 °C. A current commercial cryogenic mill can be used in this step. However, there are several kinds of mills that can be adapted with a preliminary cryogenic step, for example pin mills, knife mills, cfassificatory-separator mills, hammer mill, and others. Other advantages of the cryogenic mill are that the low temperature preserves the polyphenols stability, and also avoids oxidation by use of an inert atmosphere. These positive effects not only are applied to the polyphenols but also to the fat, which is very sensitive to the high temperature and oxygen. The total polyphenol content in the dried unfermented cocoa bean intermediate produced is in the range from 4 to 11 wt %.

Where the defatting step has been included prior to step c), it is preferable that the particle reduction step involves the use of a mill separator. There are also other milling systems which are suitable for use, for example, pin mills. A final stage of classification can also be included in this step to give a dried unfermented cocoa bean intermediate wherein at least 99wt% has a particle size of less than 120µm. Where a mill-separator is used, the final particle size is achieved by adjusting the separator speed and feeding flow. In this embodiment, the total polyphenol content of the product of step c) is in the range from 5 to 23wt%.

The process of the present invention includes a defatting step in which fat is removed from the unfermented cocoa bean. The stage at which this defatting step is included can be varied but it must be carried out at a stage prior to the extraction step d). In one embodiment of the present invention, the defatting step is carried out after step b) but prior to step c). It is in this embodiment that step c) then preferably employs the use of a mill-separator. Alternatively, in a further embodiment of the present invention, the defatting step is carried out after step c) but prior to step d). In both of these cases, several technologies can be used to reduced the fat content of the dried unfermented cocoa beans, for example, pressing, solvent extraction or supercritical fluid extraction.

In one embodiment of the present invention, the defatting step involves pressing the dried unfermented cocoa beans or intermediate using either an expeller or screw press. Preferably expeller pressing at pressures up to 50MPa is used. The resulting defatted product then has a fat content of no more than 15wt%. The defatting process provides a presscake and fat which is then removed.

One example of an expeller consists of a screw (or worm), rotating inside a cylindrical cage (barrel). The material to be pressed is fed between the screw and the barrel and propelled by rotating the screw in a direction parallel to the axis. The configuration of the screw and its shaft is such that the material is progressively compressed as it moves on, towards the discharge end of the cylinder. The gradually increasing pressure releases the oil, which flows out of the press through the slots, provided on the periphery of the barrel, while the presscake continues to move in the direction of the shaft, towards a discharge gate installed in the other extremity of the machine. Expeller presses are commonly used in the oil seed industry. The major advantage of the expeller press is that a larger amount of fat can be removed, for example, reducing the fat content of the press cake to about 8 to 9 wt%.

The temperature in the pressing process must be lower than 85°C, preferably between 60 and 75°C. The fat content in the defatted intermediates obtained is lower than 20 wt% and preferably less than 15 wt%, for example 10 wt%. The content of total polyphenols in these defatted intermediates is between 5 and 15 wt%.

Alternatively solvent extraction can be used in order to reduce the fat content. This procedure can be applied directly to the dried unfermented cocoa bean intermediates or to the defatted intermediates obtained by pressing the products obtained in steps b) or c). Preferably the solvent extraction is applied to the defatted intermediate obtained by pressing the products obtained in steps b) or c). Hexane, diethyl ether, ethyl-acetate or other non-polar solvents suitable for use in the food industry can be used as a solvent for extracting the fat. Hexane is particularly preferred. The final fat content after solvent extraction should be less than 10 wt%, less than 5 wt %, preferably less than 1 wt %, for example 0.5 wt%. The content of total polyphenols after solvent extraction is in the range from 9 to 23 wt%. After solvent extraction, the residual solvent must be removed.

Instead of using an organic solvent for the extraction of cocoa fat in a preferred embodiment of the present invention, supercritical CO₂ can be employed and/or another supercritical gas such as butane or propane. Although this process is more expensive, it avoids the problems of solvent residues in the powder and of waste disposal. The advantage of using supercritical CO₂ over organic solvents in food processing is that it leaves no residue in the final product and does not thermally damage the polyphenols in the product. The final fat content after supercritical CO₂ extraction should be less than 10wt%, preferably less than 5wt% and more preferably less than 2wt%.

An essential step of the process of the present invention is extraction of the naturally occurring polyphenols from the dry unfermented cocoa bean intermediate of step c).

The polyphenols are preferably extracted from the dry unfermented cocoa bean intermediate by a solid-liquid extraction using a solvent. The product obtained is a cocoa polyphenol extract.

For extracting the cocoa polyphenols several kinds of solvents may be used, preferably polar solvents approved by the Codex Alimentarius intended for human consumption. More preferably the use of water, ethanol or a mixture of both.

The ratio of dry unfermented cocoa bean intermediate to solvent by-products/solvent may be in the range 1:5 to 1:30 based on weight for example 1/10 wt. The temperature of extraction is preferably less than 75 °C, for example between 20 to 70 °C in order to avoid polyphenols polymerization and oxidation during the process. In the extraction, the mixture of by-products and solvents must be stirred. The extraction step is carried out in a reactor. In this respect, appropriate equipment intended for the food industry must be used, for example a 316 L reactor composed of stainless steel, or even a 304 L reactor composed of stainless steel.

The process of the present invention may include a preliminary solubilization of the by-products for extracting the polyphenols from the soluble media.

The required extraction time varies depending on the efficiency of the process. An appropriate time for an extraction step is for example, more than 1 hours, for example between 2 to 5 hours. Where possible, it is better to use multiple batch extractions for extracting polyphenols from the same sample. For example, a sample can be extracted in one step, and then the liquid (rich in polyphenols) and the extracted solids are separated by centrifugation or filtration. The moisture content in the exhausted solid usually is between 55 to 70 wt%. Optionally, this operation can be repeated, with the same conditions as previously described, with the extracted solids an additional three or four times depending on the yield of extraction desired. Preferably no more than four extractions are carried out as the increase of the yield percentage is not justified by the cost.

In a further embodiment of the present invention, the solid-liquid separation involves centrifugation. Appropriate centrifugation equipment should be used, for example a decanter for separating the remaining oil and water in the dry unfermented cocoa bean intermediate. Industrial decanters are available on the market. The centrifugation step generally produces more than 1 wt% of soluble extract, preferably more than 3 wt%, more preferably more than 5 wt%.

As another alternative, step d) can employ the use of filtration with stainless steel paper or a cellulose filter having an aperture in the range 10 to 150 µm, preferably in the range 2 to 30 µm. Suitable filters are Nutcha type filters available from Bachiller S.A. (Spain).

The content of polyphenols in the cocoa polyphenol extract obtained is in the range from 10 to 30 wt%, for example 25 wt%. The cocoa polyphenol extract obtained is in a liquid form.

In a further embodiment of the present invention, centrifugation or optional Microfiltration (MF) can be used in a second extraction step in order to remove further dissolved solids from the cocoa polyphenol extract obtained in the extraction step detailed above. In order to achieve this, centrifugation is preferred.

Centrifugation by decanter is based on the separation of two materials, where the driving mechanisms is due to a difference in the specific gravities of the two materials and an applied force derived from a change in angular velocity. Disk type centrifuges work up to 10,000 G. With this technology the dissolved solid with a particle size larger than 0.45µm can be removed from the cocoa polyphenol extract. These kind of centrifuges are also known as clarifiers or deslungers. Such centrifuges are commercially available. (i.e. Westfalia Separator Inc., Alfa Laval Inc.).

Microfiltration can be carried out both as a cross-flow separation process and as conventional dead-end filtration. Preferably in the present invention microfiltration using the cross-flow principle is used. In this method solids, bacteria and fat globules are normally the only substances rejected. Microfiltration usually operates at pressures lower than 207 kPa (30 psi).

There are several kinds of membranes that can be used for clarifying the cocoa polyphenol extract obtained in step d) using microfiltration. For example, metal membranes can be used. These membranes have a stable porous matrix, are compressed and sintered. Preferably the membranes must have precise bubble point control, pore size determined by ASTM-E-128, uniform permeability, uniform porosity, high efficiency, bi-directional flow, and should be made of 316 L stainless steel, nickel, incomel, hastelloy, and titanium), Such membranes are available commercially from Graver Technologies (i.e. Scepter) and Atech Innovations GmbH amongst others.

Other kind of membrane that can be used for microfiltration are ceramic membranes. Ceramic membranes are tubular and multi-channel. They are usually made from pure α-AL₂O₃ and a porous membrane layer from α-AL₂O₃, TiO₂ or ZrO₂. Such membranes are chemically stable (pH 0-14 using organic solvents), thermally stable (>100°C sterilization by steam), mechanically stable and chemically inert. Examples of these kind of membranes are those produced by Atech Innovations GmbH, Pall Corporation (i.e. Membralox) and others.

Polymeric membranes can also be used in microfiltration. Such membranes are usually made of polypropylene, polyvinilydene, fluoride, polytetrafluoroethylene, and polyacrilonitryle. Suitable membranes include those produced by Koch (i.e. MFK-618, MFK-601), Alfa Laval and others.

The cocoa polyphenol extract produced in step d) contains a very high proportion of the polyphenols that were present in the unfermented cocoa bean, for example, at least 80%. However, the extract is very dilute. It is therefore necessary to include a concentration step (step e)) in the process of the present invention. The purpose of including this step is to remove any excess liquid or solvent to yield a cocoa polyphenol concentrate.

In a preferred embodiment of the present invention, the cocoa polyphenol extract is concentrated using ultrafiltration. Ultrafiltration (UF) is used in order to separate the largest molecules from the cocoa polyphenol extract. UF involves using large pore membranes and fairly low pressure. Salts, sugars, organic acids, smaller peptides and polyphenols are allowed to pass, while proteins, fat and polysaccharides are rejected. For this alternative a UF can be used. This system should be capable of separating molecules larger than 10,000 dalton (i.e. membrane diameter between 0.05 to 0,1 µm).

UF membranes are usually made of ceramics, cellulosics, polysulfone and polyvinylidene fluoride among others. For example, tubular, hollow fiber, plate and spiral membranes commercially obtained. Examples of such membranes are those provided by Koch Inc. (i.e. HFM-116/100, HFM-180/513, HFK-618), Alfa Laval Inc., Inge AG Inc. (i.e. Dizzer series) and others. Such membranes operate at pressure range between 70 to 700 kPa (20 to 100 psi).

In the UF described in this invention, practically all the polyphenols pass through the membrane and only a small fraction remain in the retentate. The loss of polyphenols in the retentate should be no higher than 10wt%, preferably less than 5wt% as referred to the total dissolved solids in the cocoa polyphenol extract or clear cocoa polyphenol extract.

After the UF operation, the concentration of polyphenols in the total dissolved solids of the permeate should be up to 30wt% being preferably more than 40wt%, for example 45wt%.

Optionally the permeate obtained in the previous UF step may be passed through a very thin Nanofiltration (NF) membrane in order to eliminate the dissolved minerals from the solution. NF also allows concentration of the solution by eliminating water. NF is not as fine a separation process as Reverse Osmosis (RO), and uses membranes that are slightly more open. However membranes intended for RO can also be used in this step. NF allows small ions to pass through while rejecting larger ions and most inorganic components, depending on the size and shape of the molecule. In the present invention the NF used preferably has a molecular weight cut off higher than 50 daltons.

NF membranes are usually made of thin film composite and cellulosics. NF membranes can be hollow fiber or spiral. Examples of this kind of membranes are those provided by Koch Inc. (i.e. TFC-RO, TFC-S, TFC-SR3, SelFRO series), PCI Membrance Systems Inc. (i.e. B1 module, C10 module, AFC99), Alfa Laval Inc, and others. The membranes used for NF operate at a range between 0.001 to 0.01 µm. NF described in the present invention operate between 2000 to 5000 kPa (300 to 700psi).

In the NF described, practically all the polyphenols are retained in the retentate. The loss of polyphenols from the retentate should be no higher than 10wt%, preferably less than 5wt% as referred to the total dissolved solids present in the permeate obtained after the UF step.

After the NF operation, the concentration of polyphenols in the total dissolved solids of the retentate is in the range up to 45wt%, preferably more than 50wt%, for example 55wt%.

Importantly in the NF operation, water is removed. Therefore, the total dissolved solids in the retentate is higher than in the initial UF permeate. The total dissolved solids in the permeate is preferably higher than 5wt%, more preferably higher than 10wt%, for example as high as 20wt%.

An alternative way of concentrating the cocoa polyphenol extract of step d) is to use vacuum evaporation. Where this method is used, a vacuum dryer system is employed. This method can also be further used to concentrate the UF permeate or NF rententate where these steps have been included.

There are several kinds of industrial vacuum dryers available in the market that are suitable for concentrating the cocoa polyphenol extract e.g. horizontal dryers or vertical dryers. Preferably, the process should be carried out at a temperature less than 70 °C, more preferably less than 60 °C, for example 40 °C. The working pressure, for removing water as solvent, should be less than 30 kPa (300 mbar), preferably less than 20 kPa (200 mbar).

After removing the solvent, a product with more than 15 wt% of soluble solids is obtained, preferably with more than 25 wt% of soluble solids, for example between 45 and 65 wt% of soluble solids. The total cocoa polyphenol content in the cocoa bean concentrate obtained usually represents at least 10wt%, more preferably at least 30wt% of the total soluble solids.

The cocoa polyphenol concentrate obtained in step e) is in a liquid form. For some applications, it may be preferred that the cocoa polyphenol concentrate is in a powder form. Thus the process of the present invention can optionally include a final drying step to yield a powdered cocoa polyphenol concentrate. The powder obtained preferably has a final moisture content of less than 6wt%, more preferably less than 3wt%.

Suitable drying apparatus include a pan vacuum dryer and a spray dryer. Where a pan vacuum dryer is used, after drying, hard blocks are obtained.

Preferably, these blocks should be subjected to grinding before milling in order to reduce the particle size for making a powder. For grinding a hammer or knife type mill can be used. As for milling, a hammer mill, pin mill, clasificator-separator mill or a combination of mills can be used. The final particle size distribution is such that 99 vol% have a particle size of less than or equal to 300 µm, preferably 200 µm, more preferably 100 µm for example 75 µm.

Where a spray dryer is used several stabilizers can be used as a carrier in the drying process, for example guar gum. The final powder should preferably contain less than 10 wt% of stabilizers, preferably less than 3 wt%, for example 1 wt%.

The total cocoa polyphenol concentration in the final powder is at least 10wt% preferably at least 15wt%. Preferably no more than 5wt% of the total polyphenol content is lost in the drying and particle size reduction steps.

### Figures

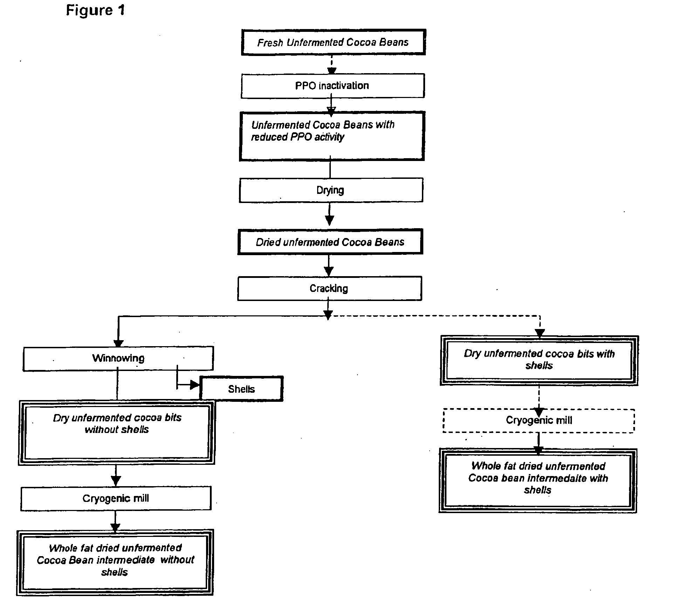

Figure 1 shows a graphical representation of examples of steps a) to c);

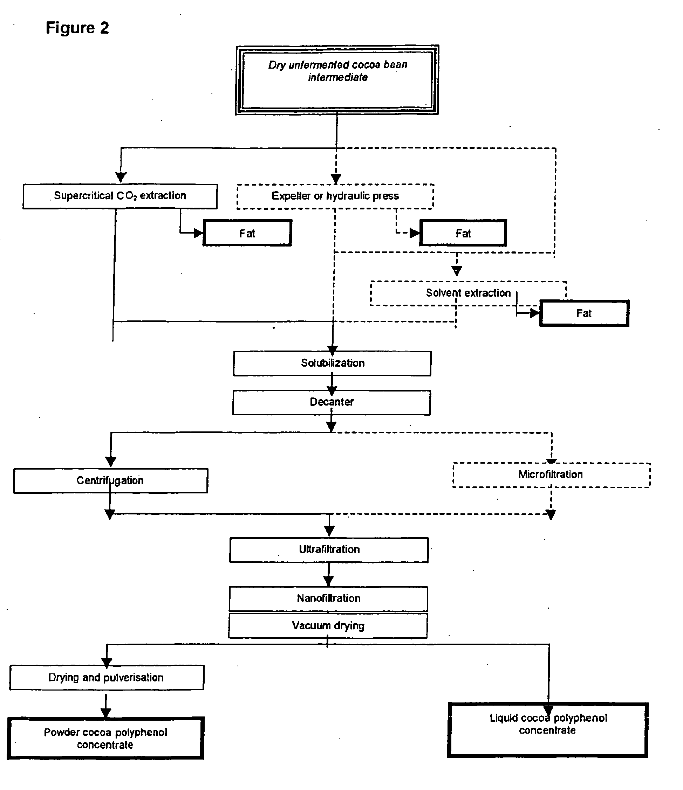

Figure 2 shows a graphical representation of steps d) and e);
Figure 3 shows the results of a statistical study into the necessary time and temperature of the blanching step a). The result shown is a 3D surface representation of the estimated conditions.
Figure 4 shows the further results of the statistical study carried out investigating step a).
Figure 5 is a surface contour representation of the results obtained from the statistical investigation into the conditions for step a).

The present invention will now be illustrated further by reference to the following examples.

### Examples

### Example 1

The enzymatic browning reaction produces the darkening of food products rich in phenolic compounds during their transportation, storage, and processing. These undesired reactions in food products produce substances responsible for disagreeable smells and tastes.

The objective was to inactivate the polyphenol oxidase by heating, specifically, by thermal treatment in water (blanching) and to study the conditions of inactivation (Time-Temperature relationship) regarding the control of the enzymatic browning reaction measured by the change of color (sensory color analysis).

Nine trials were performed under three different time and temperature combinations.
Temperatures= 75, 85 and 95 °C; Time= 5,10 and 15 minutes. 1% Catecol was used as a reagent to accelerate the browning. The browning was then measured to quantify what time and thermal treatment is necessary to optimize the inhibition of the browning reaction (melanosis) of cocoa seeds. A melanosis scale was employed to measure the level of browning within the seeds.

### Experimental Procedure:

### Raw Material: Fresh unfermented cocoa seeds.

| | |
|---|---|
| Apparatus: | Thermocouple |
| | Stopwatch |
| | Stainless steel knife |
| | Water bath with temperature control |
| | Watchglass |
| | |
| Reagents: | Catecol 1 %. |

80 grams of cocoa were submerged in 2 litres of hot water and maintained in a waterbath at the temperture and time indicated above. The cocoa was cooled as quickly as possible under a tap. The cocoa seeds were placed in a watchglass and cut in half with a knife. Drops of 1 % catecol were placed on the surface and the color of the cocoa seeds was observed. The results were recorded as illustrated in Table 1 and Table 2 below.

**Table 1. Color scale used to describe the progression of melanosis**

| Melanosis Scale | Description |
|---|---|
| 0 | No browning |
| 2 | Slight browning. Observed in some cocoa seeds. |
| 4 | Slight browning. Observed in the majority of cocoa seeds. |
| 6 | Moderate browning. Observed in the majority of cocoa seeds. |
| 8 | Strong browning. Observed in the majority of cocoa seeds. |
| 10 | Strong browning. Totally unacceptable. |

Introduction of the seeds into the water bath caused a temperature drop of 10°C. This is due to the transfer of temperature from the water to the seeds. In all cases, timing commenced when the temperature of the seeds equalled the preset temperature of the water.

**Table 2. Results of the Inactivation of PPO**

| RESULTS OF BLANCHING | | | |
|---|---|---|---|
| Time/min | 5min | 10min | 15min |
| Temperature/°C | Melanosis | Melanosis | Melanosis |
| 75°C | 6 | 6 | 8 |
| 85°C | 4 | 2 | 4 |
| 95°C | 0 | 2 | 4 |

As can be seen from the results presented in Table 2, the time and temperature that optimized the inactivation of polyphenoloxidase was at 95°C for 5 minutes. Under these conditions, a complete absence of melanosis or browning was observed in the cocoa seeds.

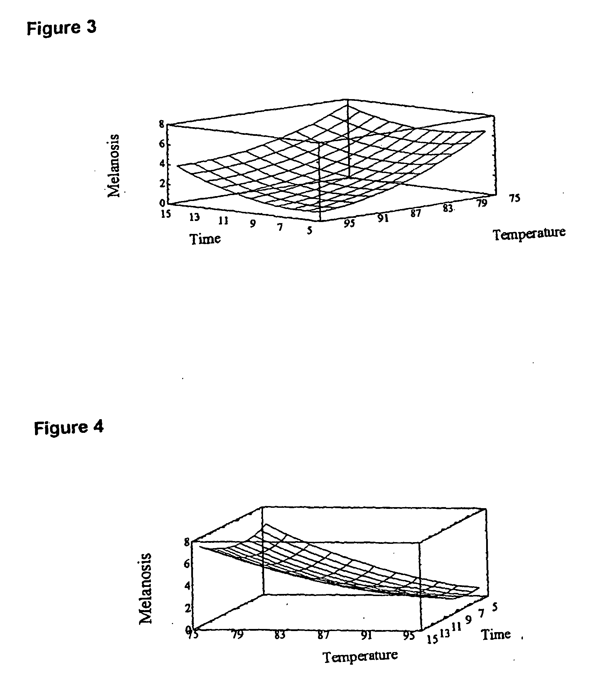

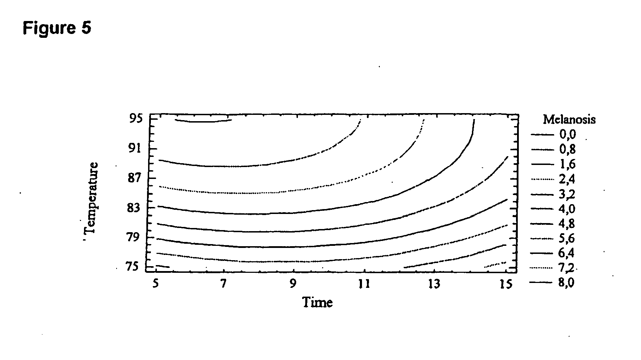

According to the results shown in Figures 3-5, the change in temperature (p<0.05) is seen to have a much larger effect on the level of melanosis than the change in time (p>0.05). A descreasing effect is seen on the level of melanosis as temperature is increased, due to the inactivation of polyphenoloxidase. Furthermore, the level of melanosis increased with an increase in time, due to non-enzymatic browning reactions or Maillard reactions that depend on elevated temperatures over prolonged time periods. Therefore, the confirmed thermal treatment necessary to avoid melanosis or browning reaction is at 95°C for 5 minutes. Longer times produce browning due to Maillard reactions. For a Melanosis scale between 0-8, the level of melanosis development at a temperature of 95°C for 5-7 minutes is 0.8. It can be concluded that thermal treatment prolonged for 2 minutes will not give a level higher then 1 on the melanosis scale.

### Example 2. Polyphenols solubilization

25 g of dry, defatted and unfermented cocoa bean intermediate containing 2.31 g of total polyphenols (9.25 wt%) was extracted initially with water in a ratio 1:15 (cocoa:water). The extraction was carried out at a temperature of 30 °C for a time period of 2.5 hours.

After the first extraction, the mixture was centrifuged. Two fractions were obtained: a liquid and exhausted solids. The exhausted solids were subjected to the preceding extraction process a further two times. The conditions were maintained the same, except that the ratio of solid:solvent in the last two extractions was 1:10.

The results of the experiment are illustrated in Table 3.

**Table 3. Results of solubilization of cocoa polyphenols**

| | Polyphenols recovered in the process (wt%) | Volume of centrifuge solution (ml) | Total solids (g) | Total polyphenols (g) |
|---|---|---|---|---|
| Extraction I | 49,11 | 335 | 4,55 | 1,13 |
| Extraction II | 18,51 | 126 | 0,86 | 0,186 |
| Extraction II | 13,46 | 133 | 0,43 | 0,104 |
| | | | | |
| Total | 81,08 | 594 | 5,84 | 1,42 |

It is clear from the results displayed in Table 3 that after the third extraction, 81.08% of polyphenols are recovered in the solution. 594 ml of solution is obtained from which 5.84 g are Total Solids (0.98 wt%), and 1.42 g are total polyphenols. The total polyphenols represents 24.27% of the total solids present in the solution.

### Example 3

Unfermented fresh cocoa beans, contain on average 50 wt% of moisture, 24 wt% of fat, 3.5 wt% of total polyphenols, 7.8 wt% of shell and 14.7 wt% of other compounds.

1000 Kg of fresh unfermented cocoa beans, were blanched in hot water at 95°C for 7 minutes to inactivate PPO enzyme. After blanching, the beans were rinsed in cool water. Next, the beans, with a moisture content of 44 wt%, were sun dried for a week to decrease the moisture content to 6 wt%. After drying 532 Kg of dry unfermented cocoa beans with greatly reduced and/or inactivated PPO activity were obtained. This by-product contained 6.57% of polyphenols.

In a next step, the particle size of the dry beans was reduced by cracking and subsequent milling. The cracking was done using a hammer-mill for obtaining bits. After cracking, the bits were milled in a cryogenic pinmill using nitrogen as a freezing media to avoid fat melting. During cryogenic milling, the by-product was frozen under - 5 °C. The final particle size distribution was 99wt% under 150µm.

The unfermented cocoa powder was defatted in order to promote dissolution, in the following operations. For de-fatting, a supercritical CO₂ extraction system was used. After the extraction, 295 Kg of unfermented defatted cocoa bean intermediate was is obtained. This by-product contained 11,86% of total polyphenols and 1% of fat. Also 237 Kg of cocoa butter obtained. This butter is suitable for human consumption.

For extracting the polyphenols by solubilization, the 295 Kg of unfermented defatted powder is mixed with 4.425 Kg of water (1:15) for the first extraction. The mixture is extracted in a reactor by agitation at 35°C for 2.5 hours. In this extraction, 60% of the initial polyphenols were in solution. After the extraction, the solids were separated using a decanter followed by a centrifuge. Two fractions were obtained. A liquid fraction of 4.291 Kg containing 1.88% Total dissloved solids TDS (80,76 Kg) from which 26% are polyphenols (21 Kg). This fraction is called the 1^{st} solution. The other fraction is made of 428,48 Kg of the semi-exhausted solids containing 50% moisture.

The exhausted solids still contain 40% of the polyphenols which were present in the unfermented cocoa beans. The 428,48 Kg of exhausted solids (50% of solids) were subjected to a second extraction step with 1.713,92 Kg of water (1:10) at the same conditions of the previous extraction. As in the previous case, two fractions were obtained. A liquid fraction of 1760,58 Kg containing 1.32% total dissolved solids (23,33 Kg) from which 30% are polyphenols (7 Kg). This fraction is called the 2^{nd} solution. The other fraction contains 381,82 Kg of exhausted solids 50% of which is moisture. The exhausted solids still contain 7 Kg of polyphenols which can be dissolved in future extractions if desired.

In the next step the solutions obtained in the 1^{st} and 2^{nd} extractions were mixed together in an intermediate tank. The mixture of both solutions represents 6052,1 Kg containing 1,71% total dissolved solids (104,1 Kg) of which 26,9% are polyphenols (28 kg).

Then, the solution was passed trough an Ultrafiltration membrane in order to remove the bigger particles (i.e. proteins, carbohydrates, etc.). The membrane used had a cut of 10.000 dalton. After the ultrafiltration two fractions are obtained, a retentate and a permeate. The retetentate weighed 605,21 Kg and contained 6,21 % total dissolved solids (37,6 Kg) from which 3,72% were polyphenols (1,4 Kg). The permeate weighed 5.446,21 Kg in solution containing 1,22% total dissolved solids (66,5 Kg) from which 40% are polyphenols (26,6 Kg).

The next step carried out was a nanofiltration performed in order to concentrate the total dissolved solids and to partially eliminate the salts. The 5.446.21 Kg of UF permeate was passed through a nanofilter. As in the previous membrane separation operation, two fractions were obtained. The permeate is made of 4.851,89 Kg containing 0,14% total dissolved solids (7 Kg), which are mainly dissolved salts. The enriched retentate weighed 595 Kg and contained 10% total dissolved solids (59,5 Kg) from which 44,7% were polyphenols (26,6 Kg).

In a next step, the 595 Kg of NF permeate was concentrated using a vertical vacuum dryer at 55 °C and 300 mbar. In the drying process, 495,04 Kg of water was removed. Finally, 99,16 Kg of liquid cocoa polyphenol concentrate containing 60% total dissolved solids was obtained (59,5 Kg). This product contains 26,82% of polyphenols (26,6 Kg).

Optionally, the liquid cocoa polyphenols concentrate can be further dried in order to obtain a powder. For this purpose, the 99,16 Kg of Liquid Polyphenol Concentrate was mixed with 5 Kg of gum guar. The mixture was then passed trough a spray dryer. Finally, 67,1 Kg of powdered cocoa polyphenol concentrate containing 4% of moisture and 39,64% of polyphenols was obtained.

## Claims

1. A process in which cocoa polyphenol concentrate is obtained comprising the steps of:
a) subjecting unfermented cocoa beans to a blanching step in water at a temperature in the range from 85-100°C for a time period in the range from 3 to 15 minutes to give unfermented cocoa beans with reduced polyphenol oxidase activity;
b) drying the unfermented cocoa beans with reduced polyphenol oxidase activity at a temperature of less than 85°C to obtain dried unfermented cocoa beans with a moisture content of no more than 15%;
c) subjecting the dried unfermented cocoa beans to a particle size reduction step to obtain dry unfermented cocoa bean intermediate whereby at least 99wt% of the product has a particle size less than or equal to 300µm;
d) extracting polyphenols from the dry unfermented cocoa bean intermediate to give a cocoa polyphenol extract and extracted solids;
e) concentrating the cocoa polyphenol extract to yield a cocoa polyphenol concentrate wherein the concentration of polyphenols present is at least 10wt%; and wherein the process further includes a defatting step which is carried out prior to step d).

2. The process according to claim 1, wherein the defatting step is carried out after step b) but prior to step c).

3. The process according to claim 1, wherein the defatting step is carried out after step c) but prior to step d).

4. The process according to claims 2 or 3, wherein in the defatting step the dried unfermented cocoa beans or cocoa bean intermediate are pressed to form a presscake and fat which is removed.

5. The process according to claim 4, wherein the pressing is carried out using an expeller press at a pressure of about 50 MPa.

6. The process according to claim 4, wherein the presscake formed is subjected to a grinding step in which the particle size is reduced to less than or equal to 5000µm.

7. The process according to claims 2 or 3, wherein in the defatting step supercritical CO₂ extraction is employed.

8. The process according to any preceding claim, wherein in the particle size reduction step, the dried unfermented cocoa beans are milled.

9. The process according to claim 8, wherein in the particle size reduction step, the dried unfermented cocoa beans are milled using a cryogenic mill.

10. The process according to any of claim 1 to 7, wherein in the particle size reduction step, the dried unfermented cocoa beans are milled using a mill-separator.

11. The process according to claim 10, wherein the milling step includes a final classification step to give a dry unfermented cocoa bean intermediate whereby at least 99wt% of the intermediate has a particle size of less than or equal to 120µm.

12. The process according to any preceding claim, wherein in step d), the polyphenols are extracted using a solvent.

13. The process according to claim 12, wherein the solvent is water or ethanol or a mixture of the two.

14. The process according to claim 12 or 13, wherein the ratio of dry unfermented cocoa bean intermediate to solvent is in the range from 1:5 to 1:30 by weight, the extraction is carried out at a temperature of no more than 75°C and during the extraction, continuous stirring is present.

15. The process according to any of claims 1 to 11, wherein in step d), the polyphenols are extracted by centrifugation or filtration.

16. The process according to any preceding claim, wherein step d) includes a preliminary solubilization step.

17. The process according to claim 16, wherein the cocoa polyphenol extract obtained in step d) is subjected to a further extraction step.

18. The process according to claim 17, wherein the further extraction step involves either centrifugation or microfiltration

19. The process according to any of claims 1 to 18, wherein in step e), the cocoa polyphenol extract is concentrated by ultra filtration to give a cocoa polyphenol permeate.

20. The process according claims 19, wherein in step e), a further step of nanofiltration is carried out after ultrafiltration to give a cocoa polyphenol retertate.

21. The process according to any of claims 1 to 18, wherein in step e), the cocoa polyphenol extract is concentrated by vacuum evaporation.

22. The process according to claims 19 or 20, wherein the cocoa polyphenol permeate and/or retentate is further concentrated by vacuum evaporation.

23. The process of any preceding claim which further comprises a final step of drying the cocoa polyphenol concentrate to obtain a powdered cocoa polyphenol concentrate.

24. The process according to claim 23, wherein the cocoa polyphenol concentrate is dried using a vacuum drier or a spray drier.

25. The process according to claim 1, which comprises an additional step of cracking and winnowing after step b) and prior to step c).

26. The process according to any preceding claim, wherein in step b), the unfermented cocoa beans with reduced polyphenol oxidase activity are dried by exposure to the sun.

27. The process according to any of claims 1 to 25, wherein in step b), the unfermented cocoa beans with reduced polyphenol oxidase activity are dried using a heated air dryer, an unheated air dryer, a commercial grain dryer, a batch fluidized bed dryer, under vacuum or a dielectric dryer.

## Patentansprüche

1. Ein Prozess, bei dem Kakao-Polyphenol-Konzentrat gewonnen wird, wobei dieser Folgendes umfasst:
a) die Unterziehung unvergorener Kakaobohnen eines Blanchierschritts in Wasser bei einer Temperatur im Bereich von 85-100°C für eine Zeitspanne im Bereich von 3 bis 15 Minuten, um unvergorene Kakaobohnen mit reduzierter Polyphenol-Oxidase-Aktivität zu erhalten;
b) die Trocknung der unvergorenen Kakaobohnen mit reduzierter Polyphenol-Oxidase-Aktivität bei einer Temperatur nicht unter 85°C, um getrocknete, unvergorene Kakaobohnen mit einem Feuchtigkeitsgehalt von nicht mehr als 15% zu erhalten;
c) die Unterziehung der getrockneten, unvergorenen Kakaobohnen eines Reduzierungsschritts der Teilchengröße, um ein trockenes, unvergorenes Kakaobohnen-Zwischenprodukt zu erhalten, bei dem mindestens 99wt% des Produktes eine Teilchengröße von weniger oder gleich 300µm besitzt;
d) die Extraktion von Polyphenolen aus dem trockenen, unvergorenen Kakaobohnen-Zwischenprodukt, um ein Kakao-Polyphenol-Extrakt und extrahierte Feststoffe zu erhalten;
e) die Konzentration des Kakao-Polyphenol-Extrakts zur Erhaltung eines Kakao-Polyphenol-Konzentrats, bei dem die Konzentration der vorliegenden Polyphenole wenigstens 10wt% beträgt; und bei dem der Prozess weiterhin einen Entfettungsschritt enthält, der vor dem Schritt d) durchgeführt wird.

2. Der Prozess nach Anspruch 1, bei dem der Entfettungsschritt nach dem Schritt b) jedoch vor dem Schritt c) durchgeführt wird.

3. Der Prozess nach Anspruch 1, bei dem der Entfettungsschritt nach dem Schritt c) jedoch vor dem Schritt d) durchgeführt wird.

4. Der Prozess nach Anspruch 2 oder 3, bei dem die getrockneten, unvergorenen Kakaobohnen oder das Kakaobohnen-Zwischenprodukt bei dem Entfettungsschritt gepresst werden, um einen Presskuchen und Fett zu bilden, das entfernt wird.

5. Der Prozess nach Anspruch 4, bei dem das Pressen durchgeführt wird, indem eine Expellerpresse bei einem Druck von ca. 50 MPa verwendet wird.

6. Der Prozess nach Anspruch 4, bei dem der gebildete Presskuchen einem Schleifschritt unterzogen wird, bei dem die Teilchengröße auf weniger oder gleich 5000 µm reduziert wird.

7. Der Prozess nach Anspruch 2 oder 3, bei dem eine überkritische CO₂ Extraktion bei dem Enfettungsschritt verwendet wird.

8. Der Prozess nach irgendeinem der vorherigen Ansprüche, bei dem die getrockneten, unvergorenen Kakaobohnen bei dem Reduzierungsschritt der Teilchengröße gemahlen werden.

9. Der Prozess nach Anspruch 8, bei dem die getrockneten, unvergorenen Kakaobohnen bei dem Reduzierungsschritt der Teilchengröße gemahlen werden, indem eine kryogene Mühle verwendet wird.

10. Der Prozess nach irgendeinem der Ansprüche 1 bis 7, bei dem die getrockneten, unvergorenen Kakaobohnen bei dem Reduzierungsschritt der Teilchengröße gemahlen werden, indem ein Mühlen-Separator verwendet wird.

11. Der Prozess nach Anspruch 10, bei dem der Mahlschritt einen abschließenden Klassifizierungsschritt einschließt, um ein trockenes, unvergorenes Kakaobohnen-Zwischenprodukt zu bieten, bei dem wenigstens 99wt% des Zwischenprodukts eine Teilchengröße von weniger oder gleich 120 µm besitzt.

12. Der Prozess nach irgendeinem der vorherigen Ansprüche, bei dem das Polyphenol bei dem Schritt d) extrahiert wird, indem ein Lösungsmittel verwendet wird.

13. Der Prozess nach Anspruch 12, bei dem es sich bei dem Lösungsmittel um Wasser oder Ethanol oder eine Mischung aus beiden handelt.

14. Der Prozess nach Anspruch 12 oder 13, bei dem das Verhältnis von trockenem, unvergorenem Kakaobohnen-Zwischenprodukt im Bereich von 1:5 bis 1:30 Massenanteil liegt, die Extraktion bei einer Temperatur von nicht über 75°C durchgeführt wird, und während der Extraktion eine ständige Durchmischung vorliegt.

15. Der Prozess nach irgendeinem der Ansprüche 1 bis 11, bei dem die Polyphenole beim Schritt d) mittels Zentrifugieren oder Filtrierung extrahiert werden.

16. Der Prozess nach irgendeinem der vorherigen Ansprüche, bei dem der Schritt d) einen vorausgehenden Schritt zur Löslichmachung beinhaltet.

17. Der Prozess nach Anspruch 16, bei dem das bei dem Schritt d) gewonnene Kakao-Polyphenol-Extrakt einem weiteren Extraktionsschritt unterzogen wird.

18. Der Prozess nach Anspruch 17, bei dem der weitere Extraktionsschritt entweder ein Zentrifugieren oder eine Mikrofiltrierung beinhaltet.

19. Der Prozess nach irgendeinem der Ansprüche 1 bis 18, bei dem das Kakao-Polyphenol-Extrakt bei dem Schritt e) mittels Ultrafiltrierung konzentriert wird, um ein Kakao-Polyphenol-Permeat zu erhalten.

20. Der Prozess nach Anspruch 20, bei dem in dem Schritt e) ein weiterer Schritt einer Nanofiltrierung nach der Ultrafiltrierung durchgeführt wird, um ein Kakao-Polyphenol-Retentat zu erhalten.

21. Der Prozess nach irgendeinem der Ansprüche 1 bis 18, bei dem das Kakao-Polhphenol-Extrakt bei dem Schritt e) mittels Vakuumaufdampfung konzentriert wird.

22. Der Prozess nach Anspruch 19 oder 20, bei dem das Kakao-Polyphenol-Permeat und/oder -Retentat weiterhin mittels Vakuumaufdampfung konzentriert wird.

23. Der Prozess nach irgendeinem der vorherigen Ansprüche, der weiterhin einen abschließenden Schritt der Trocknung des Kakao-Polyphenol-Konzentrats umfasst, um ein pulverisiertes Kakao-Polyphenol-Konzentrat zu erhalten.

24. Der Prozess nach Anspruch 23, bei dem das Kakao-Polyphenol-Konzentrat getrocknet wird, indem ein Vakuumtrockner oder Sprühtrockner verwendet wird.

25. Der Prozess nach Anspruch 1, der einen zusätzlichen Schritt der Spaltung und Ausscheidung nach dem Schritt b) und vor dem Schritt c) umfasst.

26. Der Prozess nach irgendeinem der vorherigen Ansprüche, bei dem die unvergorenen Kakaobohnen mit reduzierter Polyphenol-Oxidase-Aktivität bei dem Schritt b) getrocknet werden, indem sie der Sonne ausgesetzt werden.

27. Der Prozess nach irgendeinem der Ansprüche 1 bis 25, bei dem die unvergorenen Kakaobohnen mit reduzierter Polyphenol-Oxidase-Aktivität bei dem Schritt b) getrocknet werden, indem ein Hochtemperaturtrockner, ein Belüftungsanlage, ein gewerblicher Korntrockner, ein Wirbelschichttrockner, ein Vakuum- oder ein dielektrischer Trockner verwendet werden.

## Revendications

1. Un procédé dans lequel on obtient du concentré de polyphénol de cacao comprenant les étapes consistant à :
a) soumettre des fèves de cacao non fermentées à une étape de blanchiment dans l'eau à une température allant de 85° à 100°C pendant une période de temps allant de 3 à 15 minutes pour donner des fèves de cacao non fermentées avec une activité réduite de polyphénol oxydase ;
b) déshydrater les fèves de cacao non fermentées avec une activité réduite de polyphénol oxydase à une température inférieure à 85°C pour obtenir des fèves de cacao non fermentées déshydratées avec une teneur en humidité qui ne dépasse pas 15% ;
c) soumettre les fèves de cacao non fermentées déshydratées à une étape de réduction de la taille des particules pour obtenir un produit intermédiaire de fèves de cacao non fermentées déshydratées par laquelle au moins 99% en poids du produit a une taille de particules inférieure à ou égale à 300 µm ;
d) extraire les polyphénols du produit intermédiaire de fèves de cacao non fermentées déshydratées pour donner un extrait de polyphénol de cacao et des solides extractifs ;
e) concentrer l'extrait de polyphénol de cacao pour produire un concentré de polyphénol de cacao dans lequel la concentration de polyphénols présente est au moins de 10% en poids ; et dans lequel le procédé comprend en outre une étape de dégraissage qui a lieu avant l'étape d).

2. Le procédé selon la revendication 1, dans lequel l'étape de dégraissage a lieu après l'étape b) mais avant l'étape c).

3. Le procédé selon la revendication 1, dans lequel l'étape de dégraissage a lieu après l'étape c) mais avant l'étape d).

4. Le procédé selon les revendications 2 ou 3, dans lequel lors de l'étape de dégraissage les fèves de cacao non fermentées déshydratées ou le produit intermédiaire de fèves de cacao sont compressés pour mouler un tourteau et la graisse qui est éliminée.

5. Le procédé selon la revendication 4, dans lequel la compression est réalisée en utilisant une presse de torsion à une pression d'environ 50 MPa.

6. Le procédé selon la revendication 4, dans lequel le tourteau moulé est soumis à une étape de mouture au cours de laquelle la taille des particules est réduite à une valeur inférieure ou égale à 5000µm.

7. Le procédé selon les revendications 2 ou 3, dans lequel lors de l'étape de dégraissage l'extraction de CO₂ supercritique est employée.

8. Le procédé selon quelconque des revendications précédentes, dans lequel lors de l'étape de réduction de la taille des particules, les fèves de cacao non fermentées déshydratées sont moulues.

9. Le procédé selon la revendication 8, dans lequel lors de l'étape de réduction de la taille des particules, les fèves de cacao non fermentées déshydratées sont moulues en utilisant une meule cryogénique.

10. Le procédé selon quelconque des revendications 1 à 7, dans lequel lors de l'étape de réduction de la taille des particules, les fèves de cacao non fermentées déshydratées sont moulues en utilisant une meule classificatrice.

11. Le procédé selon la revendication 10, dans lequel l'étape de mouture comprend une étape de classification finale pour donner un produit intermédiaire de fèves de cacao non fermentées déshydratées par laquelle au moins 99% en poids du produit intermédiaire a une taille de particules inférieure ou égale à 120µm.

12. Le procédé selon quelconque des revendications précédentes, dans lequel lors de l'étape d), les polyphénols sont extraits en utilisant un solvant.

13. Le procédé selon la revendication 12, dans lequel le solvant est de l'eau ou de l'éthanol ou un mélange des deux.

14. Le procédé selon la revendication 12 ou 13, dans lequel le rapport de produit intermédiaire de fèves de cacao non fermentées déshydratées au solvant va de 1:5 à 1:30 en poids, l'extraction est réalisée à une température n'excédant pas 75°C et durant l'extraction, on agite continuellement.

15. Le procédé selon quelconque des revendications 1 à 11, dans lequel lors de l'étape d), les polyphénols sont extraits par centrifugation ou filtration.

16. Le procédé selon quelconque des revendications précédentes, dans lequel l'étape d) comprend une étape de solubilisation préliminaire.

17. Le procédé selon la revendication 16, dans lequel l'extrait de polyphénol de cacao obtenu au cours de l'étape d) est soumis à une étape d'extraction supplémentaire.

18. Le procédé selon la revendication 17, dans lequel l'étape d'extraction supplémentaire implique soit une centrifugation, soit une microfiltration.

19. Le procédé selon quelconque des revendications 1 à 18, dans lequel au cours de l'étape e), l'extrait de polyphénol de cacao est concentré par ultrafiltration pour donner un perméat de polyphénol de cacao.

20. Le procédé selon la revendication 19, dans lequel au cours de l'étape e), une étape de nanofiltration supplémentaire a lieu après l'ultrafiltration pour donner un rétentat de polyphénol de cacao.

21. Le procédé selon quelconque des revendications 1 à 18, dans lequel au cours de l'étape e), l'extrait de polyphénol de cacao est concentré par évaporation sous vide.

22. Le procédé selon les revendications 19 ou 20, dans lequel le perméat et/ou rétentat de polyphénol de cacao est de plus concentré par évaporation sous vide.

23. Le procédé de quelconque des revendications précédentes qui comprend en outre une étape finale de déshydratation du concentré de polyphénol de cacao pour obtenir un concentré de polyphénol de cacao en poudre.

24. Le procédé selon la revendication 23, dans lequel le concentré de polyphénol de cacao est déshydraté en utilisant un séchoir à vide ou un séchoir à pulvérisation.

25. Le procédé selon la revendication 1, qui comprend une étape supplémentaire de craquage et de soufflage après l'étape b) et avant l'étape c).

26. Le procédé selon quelconque des revendications précédentes, dans lequel au cours de l'étape b), les fèves de cacao non fermentées avec une activité réduite de polyphénol oxydase sont déshydratées par une exposition au soleil.

27. Le procédé selon quelconque des revendications 1 à 25, dans lequel au cours de l'étape b), les fèves de cacao non fermentées avec une activité réduite de polyphénol oxydase sont déshydratées en utilisant un séchoir à air chauffé, un séchoir à air non chauffé, un séchoir à céréales qu'on trouve dans le commerce, un séchoir à lit fluidisé discontinu, sous vide ou un séchoir diélectrique.
